# EUROPEAN PATENT SPECIFICATION

(11) **EP 1 264 610 B1**
(45) Date of publication and mention of the grant of the patent: **01.03.2006**
(21) Application number: 02009424.9
(22) Date of filing: 26.11.1997
(51) Int. Cl.: A61M 5/168

(54) **Extravasation Detection**
Erkennung von Extravasation
Détection d'épanchement

(43) Date of publication of application: 11.12.2002
(62) Divisional of application: 97949704.7
(73) Proprietor: E-Z-EM, Inc., New York 10016-2391 (US)
(72) Inventor: Goodman, Jack, Ann Arbor, MI 48103 (US); Zimmet, Arthur, Centerport, NY 11721 (US)
(74) Representative: Hibbert, Juliet Jane Grace

(56) References cited:
- EP-A- 0 784 960
- US-A- 4 010 749
- US-A- 4 877 034
- US-A- 5 334 141

## Description

This invention relates to a device for the detection of extravasation and more particularly to the detection of extravasation of ionic and non-ionic contrast media.

Extravasation or infiltration is a complication related to the use of power injectors during contrast media infection procedures. When an extravasation occurs, contrast is injected into the tissue surrounding the blood vessel, instead of into the blood vessel itself. The causes for extravasation vary, ranging from operator error in placement of the needle to physiological limitations of the blood vessel to tolerate the rate of fluid administration.

Complications related to extravasation may be quite severe and may include tissue necrosis. This may require reconstructive surgery to repair.

Presently, a method for detecting an extravasation is for the operator to visually observe it. However, by the time an extravasation is visually observable, much of the previously discussed damage may have occurred. Other known methods employ the detection of temperature variations (US-A-4 010 749) or use electromagnetic waves (US-A-4 877 034, US-A-5 334 141). Document EP-A-0 784 960 discloses an electrode assembly comprising a plurality of electrodes for electrocardiographic measurement.

Accordingly, it is an object of the present invention to provide a safe, efficient, inexpensive and reliable means for the early detection of extravasations.

A very large number of contrast media injection procedures are undertaken each year in the United States; something in the order of ten million. Less than 0.2% of these procedures result in an extravasation. Yet the absolute number is substantial because the base number is so large. The occurrence of an extravasation requires that the procedure be terminated and reinstituted. Accordingly, in a normal situation where an extravasation occurs, early detection is important from the point of view of minimizing the impact on the patient, saving time and providing a timely reinstitution of the procedure.

Although extravasation is not life-threatening, when it does occur it causes discomfort to the patient. It requires a great deal of attention from the doctor and usually means that a procedure has to be interrupted. Thus, it is important that any extravasation detection technique avoid a false indication of extravasation.

In relatively rare cases the extravasation can be quite harmful to the patient. Therefore early detection will avoid patient trauma or other injury.

The false detection of an extravasation results in terminating a procedure. Starting the procedure constitutes unnecessary trauma to the patient and expense. Therefore, any detection technique that gives a noticeable number of false indications will not be used by the doctor.

Accordingly, it is important that any detection technique to be acceptable combine an extremely small number of false indications of extravasation coupled with a reasonably high specificity to the extravasation event being detected.

The relatively large number of contrast media injections undertaken coupled with the relatively small percentage of extravasations that occur means that any procedure to be acceptable to the medical profession has to be non-invasive.

It is an accepted fact that any invasive procedure carries with it risks and trauma. They are to be avoided unless the benefit trade-off warrants such.

Thus, in order for an extravasation detection technique to be acceptable in this context, it must meet the following objectives.

First, it has to be inexpensive and be a disposable single use item.

Second, it must be relatively acceptable to the patient. Therefore, it should be non-invasive and create no pain or other patient problem.

Third, it has to be easy for the technician or doctor to use and readily fits within the procedure involved in the contrast media injection routine.

Fourth, and perhaps more importantly, it must provide next to no false indications of extravasation. A false indication would mean stopping a procedure which did not have to be stopped. Thus it follows that the technique must be specific to extravasation and non-responsive to other phenomenon such as the patient moving his or her arm.

Only a device that meets the above criteria (a) will be safe, (b) have technicians and doctors willing to use it, (c) have patients accept it and (d) have it come within the economic requirements of the institution providing the media injection procedure.

### BRIEF DESCRIPTION

The present invention relates to an extravasation detection device. The extravasation device is an electrode patch for sensing certain electrical information.

The electrode patch has a body portion which is adapted to be removably affixed to the skin of a patient. Outer and inner pairs of elongated electrodes are deployed along the body of the patch. The inner pair defines a measuring zone which is shaped and dimensioned to encompass the tip of the needle within the zone. The zone is small enough to optimize sensitivity yet large enough to facilitate placement of the patch over the needle tip. When the body of the patch is affixed to the skin of the patient and alternating electrical energy is applied to the outer electrodes, a field is provided which induces a signal in the inner electrodes, which field is a function of the impedance of the tissue of the measuring zone.

Information from the electrode patch is gathered and processed in order to calculate tissue impedance. The presence of an extravasation is determined by interpreting the tissue impedance measurement and, in that way, extravasations can be detected early. A method for determining the extravasation includes a first step of determining a pre-injection baseline measurement of the tissue impedance.

The electrode patch is affixed so that the measuring zone encompasses the tip of the needle. Energizing the outer pair of electrodes induces a signal in the inner pair of electrodes as a function of the impedance of the body tissue in the measuring zone. Tissue impedance is measured during the media injection procedure using the electrical information sensed by the inner pair of electrodes. The characteristics of the change in this impedance from the baseline impedance measurement is determined. This tissue impedance is monitored during the injection procedure. A predetermined characteristic of the change in tissue impedance indicates extravasation.

### BRIEF DESCRIPTION OF THE DRAWINGS

FIG. 1 is an overall perspective view with parts separated of the underside of the preferred embodiment, illustrating the backing paper peeling off the adhesive-backed body of the electrode patch with an open spring clip connector adjacent.
FIG. 2 is a top plan view of the electrode-patch, illustrating the conductive electrode strips within the patch.
FIG 3 is a perspective view of the lower jaw of the spring clip connector with a typical contact and hardware exploded off.

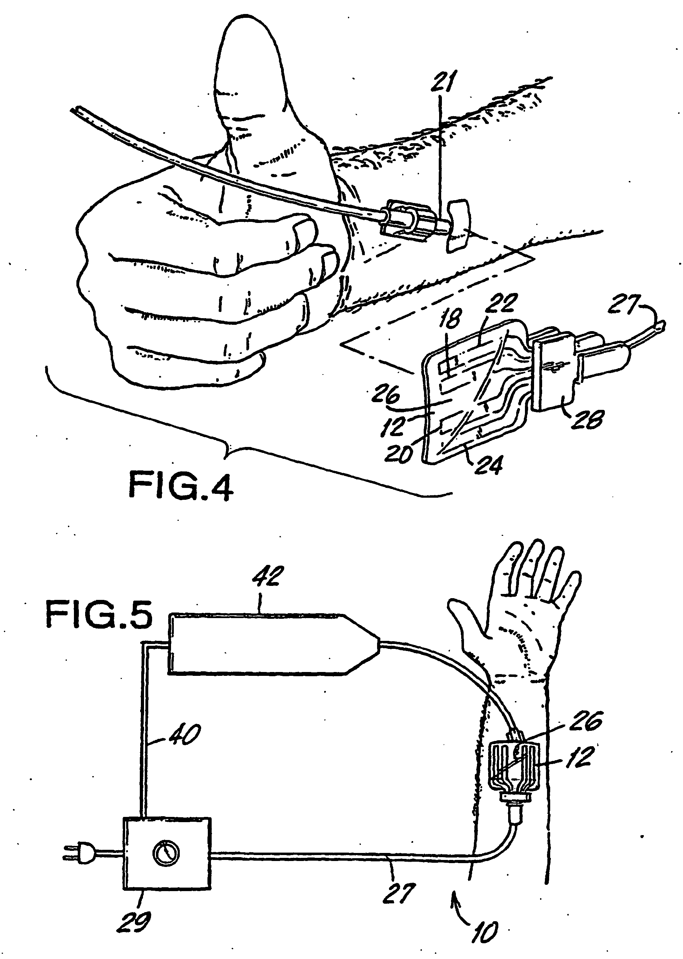

FIG 4 is a perspective view of a typical method of application, with patch and clip shown prior to placement over the point of needle insertion.

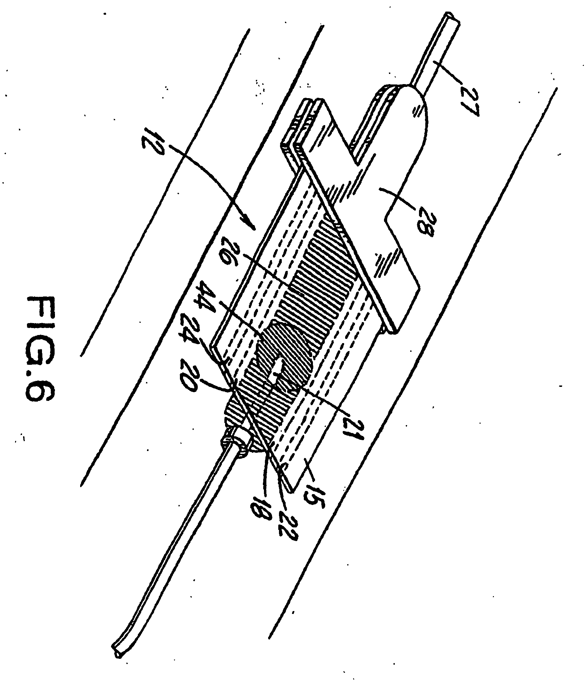

FIG 5 is a diagrammatic plan view of a typical application and apparatus hook-up.
FIG 6 is a diagrammatic plan view of the patch in place on a patient showing, in idealized form, the relation between an extravasation and the measuring zone.
FIG. 7 is a bottom plan view of a presently preferred embodiment of the patch similar to that shown in FIG. 2 except that the clear release liner or ply 68 that is the base or bottom ply is omitted from FIG. 7.
FIG. 8 is an exploded view of the FIG. 7 patch showing the plies and elements which constitute the patch.

### DESCRIPTION OF THE PREFERRED EMBODIMENT

Referring now to the drawings, the reference numeral 10 generally denotes an extravasation detection system.

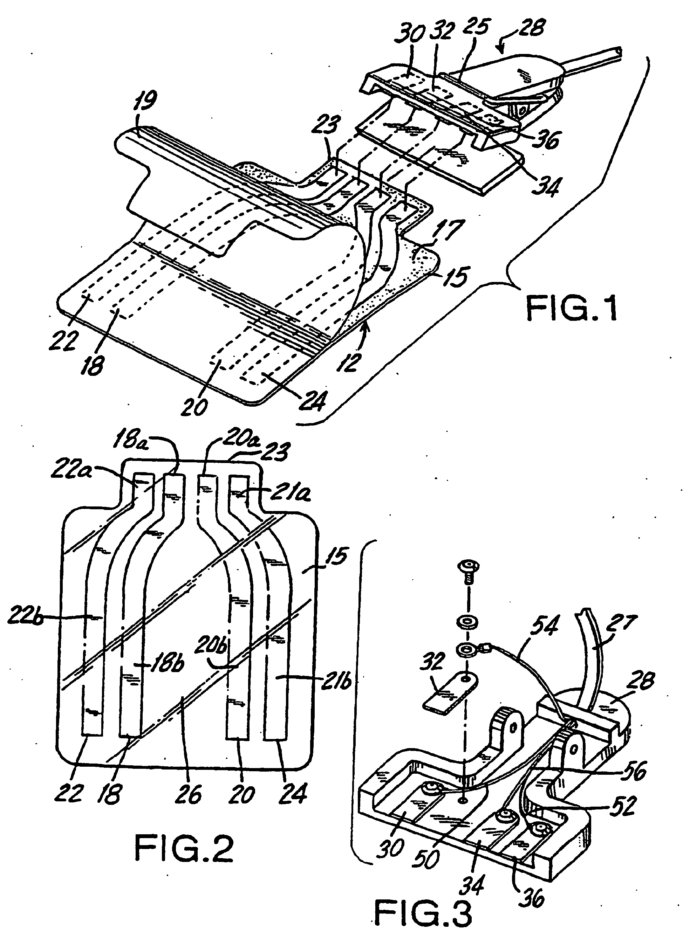

Extravasation detection system 10 includes an electrode patch 12 capable of sensing certain electrical information. Electrode patch 12, as best shown in Fig 1, includes a PVC body 15 and an adhesive backing 17. Adhesive backing 17 is protected by a clear release backing sheet 19. Electrode patch 12 is formed with four spaced apart electrodes thereon, two inner surface electrodes 18, 20 and two outer surface electrodes, 22 and 24. Between inner electrodes 18, 20 a space 26 is provided. Space 26 is shaped and dimensioned to permit a needle 21 to be placed thereunder and to optimize the sensitivity of the system for the depth of the needle tip within the tissue during a typical injection. By using adhesive backing 15, electrode patch 12 can be easily applied to, and removed from the skin.

Electrode patch 12 is provided with a coupling region 23 shaped and dimensioned to fit within a clip 28. Clip 28 is provided with electrical contacts 30, 32, 34, 36 positioned within the clip so that they can contact surface electrodes 18, 20, 22, 24 when conductor-patch 12 is placed within clip 28. In a preferred embodiment clip 28 includes a spring 25. Clip 28 has electrical leads 50, 52 which connect to a constant alternating current source of power and electrical leads 54, 56 which connects to voltage potential measuring circuitry. Clip 28 further includes a first conduit 27 housing leads 50, 52, 54, 56 which connects to a device 29 which interprets the data sensed by electrode patch 12 and a second conduit 40 which connects to a CT injector 42. Conduit 40 has capability to halt operation of injector 42 in the event an extravasation has been detected or to convey this information to injector 42.

In one embodiment, electrodes 18, 20, 22, 24 are silver/silver chloride strips. Each of the electrodes has a first relatively short vertical section 18a, 20a, 22a, 24a and a second relatively long vertical section 18b, 20b, 22b, 24b. Each electrode has a total length of about 7,62 cm (3 inches) and a width of about 0,47 cm (3/16 of an inch). Inner electrodes 18, 20 are spaced from one another by about 1,905 cm (0.75 inches), and outer electrodes 22, 24 are spaced apart by about 3,81 cm (1.5 inches).

In that embodiment, the electrode patch 12 has a length of about 7,62 cm (3 inches) and a width, at its widest point, of about 5,08 cm (2 inches).

In use, the extravasation detective system works as follows. A syringe needle 21 is introduced into the patient's vasculature. The release backing 19 is removed from the patch body 15 and the electrode patch 12 is then adhered to the patient's skin using adhesive backing 17. As heretofore mentioned, patch 12 is positioned such that the needle tip is covered by the space 26. Electrode patch 12 is clipped into clip 28 via coupling region 23 so that surface electrodes 18, 20, 22, 24 are in contact with electrical contacts 30, 32, 34, 36. Clip 28 is then connected through conduit 27 to impedance monitoring and interpreting circuitry in device 29. The provision of the short vertical sections allows use of one clip for all electrical connections without compromising the spacing of the surface electrodes in the measurement area 26 of the electrode patch 12 where measurements are being made.

Preliminary data is collected to determine the tissue impedance before any injection is made. An injection is then started using injector 42. Continuous calculations of tissue impedance are made during the injection procedure. An extravasation is deemed to have occurred if during the injection procedure the impedance change shows a fairly consistent slope of at least plus or minus 0.5 ohms per second when material is being infused into the vasculature at a rate of more than 0.25 milliliters per second. It is contemplated that, in certain embodiments, if it is determined that such an extravasation has occurred, there will be an automatic stop mechanism to cease the injection of the media, via conduit 40 or in the alternative some visual or other type of warning signal. Ionic contrast media has a lower impedance than tissue and will cause a decrease in tissue impedance during an extravasation. Non-ionic contrast media has a higher impedance than tissue and will cause an increase in tissue impedance during an extravasation.

In order to have the appropriate data derived from the electrode patch 12 a constant alternating current is applied to the two outer electrodes 22, 24. The current and frequency used is about 200 micro amperes sinusoidal at 20 kilohertz. Inner electrodes 18, 20 provide measurement of voltage potential.

Device 10 provides a method of detecting extravasations. The method includes the steps of determining a pre-injection of baseline measurement for tissue impedance. It also involves the step of determining the amount of change in tissue impedance which indicates an extravasation.

Further, the method involves the step of monitoring tissue impedance during an injection procedure to ascertain if the amount of change previously determined indicates an extravasation has occurred.

The aforementioned method, and system 10, has been used in conjunction with injections of both ionic and non-ionic contrast media to determine the existence of extravasation.

The slope change which is indicative of an extravasation was derived from a series of tests done on animals. Animals were intravenously injected, with both ionic and non-ionic contrast media. Prior to each injection, a measurement of tissue impedance was made and during the course of the injections continuous measurements of tissue impedance were made. It was found that when the injections were intravenous (no extravasation) there was very little change in impedance over time. A second series of ionic and non-ionic contrast media were also made.

These injections were deliberately made out of the vasculature to simulate an extravasation. During these injections, a substantial change in tissue impedance occurred almost instantaneously. Tissue impedance was plotted as a function of time to determine the slope change indicative of an extravasation.

Set forth below in Table 1 is a summary of four studies done on dogs in the aforementioned manner. Tables 2-5 are the underlying studies summarized in Table 1.

**Table 1 Summary of Data From Five Dogs**

| Variable | I.V. Infusion Ionic Media | Extravasation Ionic Media | LV. Infusion Non-Toxic Media | Extravasation Non-Ionic Media |
|---|---|---|---|---|
| Resting Impedance | 36 Ohms | 35.2 Ohms | 29.4 Ohms | 32.6 Ohms |
| Slope | 9.1% per minute | -163% per minute | 20.0% per minute | 172% per minute |

**TABLE 2 Results of Intravenous Injection of Ionic Contrast in 5 Dogs**

| | Dog 1 | Dog 2 | Dog 3 | Dog 4 | Dog 5 | Mean (S.D.) |
|---|---|---|---|---|---|---|
| Leg | L | R | R | L | R | |
| Baseline Resist- ance | 30 Ohms | 47 Ohms | 29 Ohms | 36 Ohms | 38 Ohms | 36.0 (7.2) |
| Injected Volume | 20cc | 10cc | 10cc | 15cc | 50cc | 21.0cc (16.7cc) |
| %ΔZ/ml | 0.05 | 0.30 | 0.16 | 0.13 | 0.08 | 0.14(0.10) |
| ΔOhms/ml | 0.015 | 0.14 | 0.05 | 0.05 | 0.03 | 0.06 (0.05) |
| %ΔZ/min | 5.4 | 18.0 | 10.0 | 7.5 | 4.6 | 9.1 (5.4) |

**TABLE 3 Results of Extravasation of Ionic Contrast in 5 Dogs**

| | Dog 1 | Dog 2 | Dog 3 | Dog 4 | Dog 5 | Mean (S.D.) |
|---|---|---|---|---|---|---|
| Leg | L | R | R | L | R | |
| Baseline Resistance | 30 Ohms | 47 Ohms | 30 Ohms | 37 Ohms | 32 Ohms | 35.2(7.2) |
| Injected Volume | 10cc | 6cc | 3cc | 6cc | 5cc | 6.0cc (2.5cc) |
| %ΔZ/ml | -2.3 | -4.0 | -1.3 | -2.0 | -4.0 | -2.7(1.2) |
| ΔOhms/ml | -0.69 | -1.9 | -0.38 | -0.74 | -1.28 | -1.0 (0.6) |
| %ΔZ/min | -140.0 | -240.0 | -75.0 | -120.0 | -240.0 | -163.0(74) |

**TABLE 4 Results of Intravenous Injection of Non-Ionic Contrast in 5 Dogs**

| | Dog 1 | Dog 2 | Dog 3 | Dog 4 | Dog 5 | Mean (S.D.) |
|---|---|---|---|---|---|---|
| Leg | R | L | L | R | L | |
| Baseline Resistance | 30 Ohms | 24 Ohms | 27 Ohms | 35 Ohms | 31 Ohms | 29.4(4.2) |
| Injected Volume | 10cc | 10cc | 6cc | 4cc | 10cc | 8.0cc (2.8cc) |
| %ΔZ/ml | 0.30 | 0.43 | 0.32 | 0.11 | 0.50 | 0.33 (0.15) |
| ΔOhms/ml | 0.09 | 0.10 | 0.09 | 0.04 | 0.16 | 0.10 (0.4) |
| %ΔZ/mim | 18.0 | 26.0 | 19.2 | 6.7 | 30.0 | 20.0(8.9) |

**TABLE 5 Results of Extravasation of Non-Ionic Contrast in 5 Dogs**

| | Dog 1 | Dog 2 | Dog 3 | Dog 4 | Dog 5 | Mean (S.D.) |
|---|---|---|---|---|---|---|
| Leg | R | L | L | R | L | |
| Baseline 30 Resistance | Ohms | 24 Ohms | 28 Ohms | 32 Ohms | 49 Ohms | 32.6 (9.6) |
| Injected Volume | 5cc | 5cc | 3cc | 4cc | 3cc | 4.0cc (1.0cc) |
| %ΔZ/ml | 1.4 | 3.0 | 4.0 | 1.9 | 4.1 | 2.9(1.2) |
| ΔOhms/ml | 0.41 | 0.72 | 1.12 | 0.60 | 2.0 | 1.0(0.6) |
| %ΔZ/min | 81.6 | 180.0 | 240.0 | 112.5 | 246.0 | 172.0 (74.0) |

Device 10 and the method associated therewith, although thus far only used to determine extravasations of ionic and non-ionic contrast media, may be useful to determine extravasations of other types of injectable fluids.

One value of an extravasation detection system employing au electrode patch is that it involves a non-invasive procedure. Another important consideration is that the electrode configuration adequately encompasses and responds to the extravasation.

During a procedure when the needle is in place within a vein, one cannot visualize exactly where the needle tip is. Since the extravasation occurs at the needle tip, one cannot be certain of where that extravasation will precisely occur along the path of the blood vessel. This electrode patch with its elongated measuring zone 26 (between the pickup electrodes 18 and 20 of FIG. 2) provides the required sensing area.

Furthermore, it is important that these sensing electrodes 18 and 20 have the opening 26 between them that is shown in FIG. 2 so that the zone under that space 26 that is within the patient's body will be sensed if an extravasation occurs.

These elongated sensing electrodes 18, 20 and parallel elongated energizing current electrodes 22, 24 provide the configuration necessary to reliably pickup an extravasation where it occurs. This is illustrated in FIG. 6. Specifically, this sensitivity occurs because applicant's structure assures placement of the electrodes 18, 20, 22, 24 around the point where the needle 21 enters the skin. Thus, the extravasation 44 is substantially centered in the measurement zone that is subtended by the inner electrodes 18, 20. In general, the extravasation will be picked up within ten to twenty ccs of extravasation.

It is the geometric configuration set forth in the above referenced application which meets the objective of providing substantial assurance that an extravasation will be detected yet nearly completely avoid providing a false indication of extravasation.

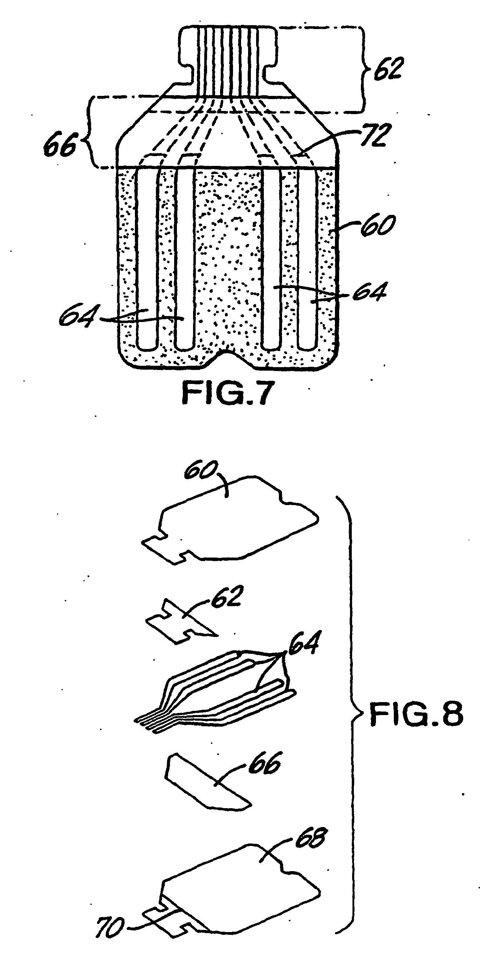

FIGs. 7 and 8 illustrate a presently preferred embodiment of the patch. As best seen in the exploded view of FIG. 8, the top of the patch is a clear vinyl ply 60. This ply 60, has on the surface facing the patient, an adhesive which serves to hold the electrodes and to adhere the patch to the patient. Under this vinyl ply 60, there is a reinforcement ply 62 that provides rigidity for the end of the patch that is to be held by the clamp 28 (see FIG. 1). Just below the reinforcement 62, and in large part in contact with and held by the adhesive side of the ply 60 is the set of four electrodes 64. A discussed in connection with FIG. 2, each electrode has an elongate portion. These elongate portions are the active portions for providing the field and for picking up the signal. These electrodes 64 are essentially similar to the electrode arrangement shown in FIG. 1. The patient side of each electrode has a hydrogel coating to assure good contact against the patient's skin. Since this hydrogel is conducting, it is important that the hydrogel coating only be on the electrode and not on any of the surfaces between the electrode since such would tend to short out the signals involved. A clear insulating tape 66 along the short portions of the electrodes has the important function of minimizing interaction between the short portion of the electrodes and the patient so that it is the long portion of the electrodes 64 which are the effective energization and pick up electrodes. Finally, there is the clear release liner 68 having a perforated line 70 that provides the base liner of the patch. As shown in FIG 1, the release liner (which is the liner 19 in FIG. 1) can be bent back initially so that the patch can be placed into the clamp 28 before it is put into use. Then when it is put into use, the main portion of the liner 68 can be removed by ripping it at the perforation line 70 so that the electrodes 64 can be placed against the patient's skin. The patient side of the vinyl layer 60 has the pressure sensitive adhesive that will adhere the patch firmly to the patient's skin.

FIG. 7 shows the assembly of the FIG. 8 plies with the clear vinyl ply omitted. The overall dimensions are about 9,4 cm by 5,84 cm (3.7 inches by 2.3 inches). The electrodes 64 are each about 0,51 cm (0.2 inches) wide and the elongate portions are about 5,08 cm (two inches). The hydrogel coating in the electrodes 64 ends at the line 72. The spacing between the inboard edges of the inner electrodes is about 1,78 cm (0.70 inches) and the spacing between the inboard edges of the outer pair of electrodes is about 3,81 cm (1.5 inches).

## Claims

1. A device for use with a non-invasive detection system to detect extravasation when fluid is delivered into a patient's vascular system, comprising:
a) an outer pair of elongated electrodes (22, 24) and an inner pair of elongated electrodes (18, 20) deployed along a patch (12);
b) said inner pair of electrodes being spaced from one another on either side of a centre line, said inner pair defining a measuring zone (26), said measuring zone (26) being shaped and dimensioned to encompass within said zone an end of a channel (21) inserted in the patient's vascular system for delivering fluid into the vascular system, said zone (26) being spaced to provide adequate sensitivity and long enough to facilitate placement of the end of the channel (21) within the measuring zone (26);
c) each of said outer pair of electrodes being outward, relative to said centre line, of a respective one of said inner electrodes (18, 20);
d) means for providing energization of said outer electrodes for providing a field which induces a signal in said inner electrodes that is a function of the impedance of the tissue in said measuring zone.

2. A device as claimed in claim 1, wherein said zone is small enough to optimize sensitivity.

3. A device as claimed in claim 1, wherein said zone is of uniform sensitivity and sized to encompass the end of the channel (21).

4. The device of any preceding claim wherein the electrodes are substantially the same length.

5. The device of any preceding claim wherein each electrode (18, 20, 22, 24) is about 7.6 cm (3 inches) in length, and about 0.48 cm (3/16^{th} of an inch) wide.

6. The device of any preceding claim, wherein the electrodes (18, 20, 22, 24) are silver/silver chloride strips.

7. The device of any preceding claim wherein a hydrogel material is placed on the electrodes (18, 20, 22, 24).

8. The device of any preceding claim wherein the length of the electrodes (18, 20, 22, 24) are parallel relative to each other.

9. The device of any preceding claim, wherein said patch is adapted to be affixed to a patient's skin.

10. The device of any preceding claim, wherein the end of a channel inserted in the patient's vascular system is a needle tip (21).

11. The device of any preceding claim, wherein each of the electrodes includes a coupling region (23) capable of being connected to a clip (28) having electrical contacts (30, 32, 34, 36).

12. The device of any preceding claim, wherein the inner pair of electrodes (18, 20) are separated from each other to encompass a vicinity around the end of the channel and the measuring zone (26) being sized to encompass the vicinity around the end of the channel so that extravasation occurring in said vicinity is within the measuring zone.

13. The device of any preceding claim, wherein the means for providing energization is adapted to supply a current in the micro ampere range at a frequency of about 1 kilo-hertz to 20 kilo-hertz.

14. The device of any preceding claim, wherein the means for providing energization is adapted to supply a current in the micro ampere range at a frequency of about 20 kilo-hertz.

15. The device of any preceding claim, wherein the patch (21) is about 5cm (two inches) in width and about 5cm (two inches) in length.

## Patentansprüche

1. Gerät zum Verwenden in einem nicht-invasiven Erkennungssystem, um Extravasation zu erkennen, wenn Flüssigkeit in das Gefäßsystem eines Patienten abgegeben wird, welches aufweist:
a.) ein äußeres Paar langgestreckter Elektroden (22, 24) und ein inneres Paar langgestreckter Elektroden (18, 20), die entlang eines Pflasters (12) ausgebreitet sind;
b.) das innere Elektrodenpaar, das auf beiden Seiten einer Mittellinie voneinander beabstandet ist, wobei das innere Paar einen Meßbereich (26) begrenzt, wobei der Meßbereich (26) so geformt und dimensioniert ist, daß er innerhalb des Bereichs ein Ende eines Kanals (21) umschließt, welches in das Vaskularsystem eines Patienten eingesetzt ist, um Flüssigkeit in das Vaskularsystem abzugeben, wobei der Bereich (26) beabstandet ist, um hinreichende Empfindlichkeit zu bieten und lang genug ist, um das Anordnen des Endes des Kanals (21) innerhalb des Meßbereich (26) zu ermöglichen;
c.) jede Elektrode des äußeren Paars befindet sich, bezogen auf die Mittellinie, außerhalb der jeweiligen der inneren Elektroden (18, 29);
d.) Mittel zum unter Strom setzen der äußeren Elektroden zum bilden eines Feldes, welches ein Signal in die inneren Elektroden induziert, welches eine Funktion der Impedanz des Gewebes im Meßbereich ist.

2. Gerät nach Anspruch 1, wobei der Bereich klein genug ist, die Empfindlichkeit zu optimieren.

3. Gerät nach Anspruch 1, wobei der Bereich von einheitlicher Empfindlichkeit ist und bemessen ist, um das Ende des Kanals (21) einzuschließen.

4. Gerät nach einem der vorhergehenden Ansprüche, wobei die Elektroden im wesentlichen die gleiche Länge aufweisen.

5. Gerät nach einem der vorhergehenden Ansprüche, wobei jede Elektrode (18, 20, 22, 24) etwa 7,6 cm (3 Zoll) lang und etwa 0,48 cm (3/16 Zoll) breit ist.

6. Gerät nach einem der vorhergehenden Ansprüche, wobei die Elektroden (18, 20, 22, 24) Silber- / Silberchlorid-Streifen sind.

7. Gerät nach einem der vorhergehenden Ansprüche, wobei ein Hydrogel-Material an den Elektroden (18, 20, 22, 24) angeordnet ist.

8. Gerät nach einem der vorhergehenden Ansprüche, wobei die Längsrichtung der Elektroden (18, 20, 22, 24) im Bezug zueinander parallel sind.

9. Gerät nach einem der vorhergehenden Ansprüche, wobei das Pflaster geeignet ist, um an der Haut des Patienten angebracht zu werden.

10. Gerät nach einem der vorhergehenden Ansprüche, wobei das in das Gefäßsystem des Patienten eingeführte Ende des Kanals eine Nadelspitze (21) ist.

11. Gerät nach einem der vorhergehenden Ansprüche, wobei jede der Elektroden einen Anschlußbereich (23) aufweist, der geeignet ist, um an eine Klemme (28), welche elektrische Kontakte (30, 32, 34, 36) aufweist, angeschlossen zu werden.

12. Gerät nach einem der vorhergehenden Ansprüche, wobei das Paar der inneren Elektroden (18, 20) voneinander getrennt ist, um einen Nachbarbereich um das Ende des Kanals und den Meßbereich (26) einzuschließen, der bemessen ist, um den Nachbarbereich um das Ende des Kanals herum einzuschließen, so daß Extravasation, die in dem Nachbarbereich auftritt, sich innerhalb des Meßbereichs befindet.

13. Gerät nach einem der vorhergehenden Ansprüche, wobei das Mittel zum unter Strom setzen geeignet ist, einen Strom im Mikroampere-Bereich bei einer Frequenz von etwa 1 Kiloherz bis 20 Kiloherz zu liefern.

14. Gerät nach einem der vorhergehenden Ansprüche, wobei das Mittel zum unter Strom setzen geeignet ist, einen Strom im Mikroampere-Bereich bei einer Frequenz von etwa 20 Kiloherz zu liefern.

15. Gerät nach einem der vorhergehenden Ansprüche, wobei das Pflaster (21) etwa 5 cm (zwei Zoll) breit und etwa 5 cm (zwei Zoll) lang ist.

## Revendications

1. Dispositif destiné à être utilisé avec un système de détection non invasif pour détecter un épanchement lorsqu'un fluide est administré dans le système vasculaire d'un patient, comprenant :
a) une paire externe d'électrodes allongées (22, 24) et une paire interne d'électrodes allongées (18, 20) déployées le long d'un patch (12) ;
b) les électrodes de ladite paire interne étant espacées l'une de l'autre de part et d'autre d'une ligne centrale, ladite paire interne définissant une zone de mesure (26), ladite zone de mesure (26) étant configurée et dimensionnée de manière à englober au sein de ladite zone une extrémité d'un conduit (21) introduit dans le système vasculaire d'un patient afin d'administrer un fluide dans le système vasculaire, ladite zone (26) étant étendue afin de procurer une sensibilité adéquate et suffisamment longue afin de faciliter le placement de l'extrémité du conduit (21) dans la zone de mesure (26) ;
c) chaque électrode de ladite paire externe étant placée à l'extérieur, par rapport à ladite ligne centrale, d'une électrode respective desdites électrodes internes (18, 20) ;
d) un moyen d'alimentation desdites électrodes externes destiné à procurer un champ qui induit un signal dans lesdites électrodes internes qui est fonction de l'impédance du tissu dans ladite zone de mesure.

2. Dispositif selon la revendication 1, **caractérisé en ce que** ladite zone est suffisamment petite pour optimiser la sensibilité.

3. Dispositif selon la revendication 1, **caractérisé en ce que** ladite zone présente une sensibilité uniforme et est dimensionnée de manière à englober l'extrémité du conduit (21).

4. Dispositif selon l'une quelconque des revendications précédentes, **caractérisé en ce que** les électrodes ont sensiblement la même longueur.

5. Dispositif selon l'une quelconque des revendications précédentes, **caractérisé en ce que** chaque électrode (18, 20, 22, 24) présente une longueur d'environ 7,6 cm (3 pouces) et une largeur d'environ 0,48 cm (3/16èmes d'un pouce).

6. Dispositif selon l'une quelconque des revendications précédentes, **caractérisé en ce que** les électrodes (18, 20, 22, 24) sont des bandes d'argent ou de chlorure d'argent.

7. Dispositif selon l'une quelconque des revendications précédentes, **caractérisé en ce qu'**un matériau d'hydrogel est placé sur les électrodes (18, 20, 22, 24).

8. Dispositif selon l'une quelconque des revendications précédentes, **caractérisé en ce que** les électrodes (18, 20, 22, 24) sont parallèles les unes par rapport aux autres sur leur longueur.

9. Dispositif selon l'une quelconque des revendications précédentes, **caractérisé en ce que** ledit patch est apte à être fixé sur la peau d'un patient.

10. Dispositif selon l'une quelconque des revendications précédentes, **caractérisé en ce que** l'extrémité d'un conduit introduit dans le système vasculaire d'un patient est la pointe d'une aiguille (21).

11. Dispositif selon l'une quelconque des revendications précédentes, **caractérisé en ce que** chaque électrode comprend une zone de raccordement (23) pouvant être connectée à une pince (28) présentant des contacts électriques (30, 32, 34, 36).

12. Dispositif selon l'une quelconque des revendications précédentes, **caractérisé en ce que** les électrodes de la paire interne (18, 20) sont séparées l'une de l'autre de manière à englober une zone aux abords de l'extrémité du conduit et la zone de mesure (26) étant dimensionné de manière à englober la zone aux abords de l'extrémité du conduit de sorte que l'épanchement se produisant dans ladite zone se trouve à l'intérieur de la zone de mesure.

13. Dispositif selon l'une quelconque des revendications précédentes, **caractérisé en ce que** le moyen d'alimentation est apte à procurer un courant de l'ordre du microampère à une fréquence d'environ 1 kilohertz à 20 kilohertz.

14. Dispositif selon l'une quelconque des revendications précédentes, **caractérisé en ce que** le moyen d'alimentation est apte à procurer un courant de l'ordre du microampère à une fréquence d'environ 20 kilohertz.

15. Dispositif selon l'une quelconque des revendications précédentes, **caractérisé en ce que** le patch (21) présente une largeur d'environ 5 cm (deux pouces) et une longueur d'environ 5 cm (deux pouces).
